**Europäisches Patentamt**

(19) **European Patent Office**

**Office européen des brevets**

(11) Veröffentlichungsnummer: **0 181 388**
**B1**

(12) **EUROPÄISCHE PATENTSCHRIFT**

(45) Veröffentlichungstag der Patentschrift:
**29.03.89**

(21) Anmeldenummer: **85902529.8**

(22) Anmeldetag: **18.05.85**

(86) Internationale Anmeldenummer:
**PCT/EP 85/00235**

(87) Internationale Veröffentlichungsnummer:
**WO 85/05455 (05.12.85 Gazette 85/26)**

(51) Int. Cl.⁴: **G 01 S 17/10,** G 01 S 7/48,
A 61 B 1/00

(54) **OPTOELEKTRISCHES ENTFERNUNGSMESSGERÄT MIT EINER OPTISCHEN MESSONDE.**

(30) Priorität: **24.05.84 DE 3419320**

(43) Veröffentlichungstag der Anmeldung:
**21.05.86 Patentblatt 86/21**

(45) Bekanntmachung des Hinweises auf die Patenterteilung:
**29.03.89 Patentblatt 89/13**

(84) Benannte Vertragsstaaten:
**AT BE CH FR GB IT LI NL SE**

(56) Entgegenhaltungen:
**EP-A-0 076 232**
**EP-A-0 092 369**
**EP-A-0 093 437**
**EP-A-0 135 423**
**WO-A-85/01117**
**US-A-4 325 638**

**Optics Letters, vol. 9, no. 2, February 1984, New York (US), H. Nishihara et al.: "Use of laser diode and optical fiber for a compact laser-doppler velocimeter", pp. 62-64**

(73) Patentinhaber: **SCHWARTE, Rudolf, Kreuztaler Strasse 56, D-5902 Netphen- Dreistiefenbach (DE)**

(72) Erfinder: **SCHWARTE, Rudolf, Kreuztaler Strasse 56, D-5902 Netphen- Dreistiefenbach (DE)**

(74) Vertreter: **Pürckhauer, Rolf, Friedrich- Ebert- Strasse 27 Postfach 10 09 28, D-5900 Siegen 1 (DE)**

## Beschreibung

Die Erfindung betrifft ein optoelektronisches Entfernungsmeßgerät mit einem eine Laserdiode enthaltenden Lichtsender zur Erzeugung kurzer Lichtimpulse, einem mit einer Photodiode ausgestatteten Photoempfänger, einer Meßsonde, welche eine Linsenoptik, einen die Lichtimpulse der Linsenoptik zuführenden Sendelichtleiter sowie einen Empfangslichtleiter zur Weiterleitung der nach Reflexion an einem Zielobjekt über die Linsenoptik empfangenen Lichtimpuls auf die Photodiode aufweist, und einer Auswerteschaltung, welche einen Taktgeber sowie einen Zähler zur Zählung der vom Taktgeber gelieferten Impulse während vorgegebener, durch die Aussendung und den Empfang der Lichtimpulse begrenzter Zeitintervalle enthält.

Bei einem aus der EP-A-0 076 232 bekannten optoelektrischen Entfernungsmeßgerät dieser Art wird einerseits die Laufzeit der von der Laserdiode emittierten, von einem Zielobjekt reflektierten und der Photodiode empfangenen Lichtimpulse (Zielimpulse) und andererseits die Laufzeit der von der Laserdiode emittierten, jedoch über die Lichtweichen und den kurzen Weg zur Photodiode gelangenden Referenzimpulse bestimmt und zur Ermittlung der Entfernung ausgewertet. Dabei löst der zuerst eintreffende Referenzimpuls nach einer optoelektrischen Umsetzung in einem Zeitkomparator ein Torzeitsignal aus, das durch den später eintreffenden, über das Zielobjekt laufenden Zielimpuls abgeschaltet wird. Während der gesteuerten Torzeit werden die von einem Quarzoszillator fortlaufend erzeugten Taktimpulse (150 MHz) einem Zähler aufgeschaltet. Die gezählten Pulse werden gruppenweise einem Prozessor zugeführt, welcher durch Sortierung und Mittelwertbildung die zu bestimmende Entfernung ermittelt. Bei den bekannten Meßgeräten dieser Art ist für die Sendeoptik und Empfangsoptik jeweils eine eigene Linse vorgesehen. Diese nebeneinander am Gerät angeordneten Linsen haben den Nachteil, daß der von der Sendelinse gegen ein nichtkooperatives Zielobjekt gerichtete Lichtimpuls bei Entfernungen unter etwa 2 m nicht in die Empfangslinse reflektiert wird. Erst bei einem Abstand von mehr als 2 m beginnt eine im Querschnitt linsenförmige Überlappung der optischen Sende- und Empfangskanäle, wobei jedoch die im Nahbereich vorhandene Teilüberlappung zu erheblichen Meßfehlern führt. Für kleinere Entfernungen sind diese doppellinsigen Meßgeräte daher ungeeignet. Ein weiterer Nachteil besteht auch darin, daß die Sende- und Empfangsoptik unmittelbar am Gerätegehäuse angeordnet ist, welches auch die Bauteile zur Erzeugung der Lichtimpulse, zur Umwandlung der empfangenen Lichtsignale in elektrische Impulssignale und zur Verarbeitung und Auswertung der elektrischen Impulssignale enthält. Erhebliche Schwierigkeiten bereitet auch die von der Laserdiode ausgehende und über einen aus Glasfaser bestehenden Sende-Lichtleiter zur Sendelinse übertragene inhomogene Phasenfront der Lichtimpulse.

Demgegenüber liegt der Erfindung die Aufgabe zugrunde, ein optoelektrisches Entfernungsmeßgerät der gattungsgemäßen Art derart auszubilden, daß eine möglichst genaue Entfernungsmessung auch im Nahbereich möglich ist.

Diese Aufgabe wird erfindungsgemäß durch die in Anspruch 1 angegebenen Merkmale gelöst.

Durch die in Anspruch 1 definierte Ausbildung des Entfernungsmeßgeräts liegen der optische Sende- und Empfangskanal im Linsenbereich unmittelbar nebeneinander, nur durch die Ebene eines äußerst dünnen Spiegels getrennt, so daß eine Hälfte der Linse dem Sendekanal und die andere Hälfte dem Empfangskanal zugeordnet ist. Die im Bereich des Brennpunktes endenden Sende- und Empfangs-Lichtleiter erlauben ein Ausmessen bis an die Linse heran. Die optoelektrische Entfernungsmessung ist daher auch zur Füllstandanzeige von Tankbehältern, Schüttgutbehältern u. dgl. bestens geeignet. Auch überschneiden sich nicht die Ränder der Sende- und Empfangskeulen, sondern die Überschneidung geschieht an der im Querschnitt breitesten Stelle beider Keulen und ist somit für die Phasenlaufzeiten repräsentativ. Das Halblinsensystem hat den weiteren Vorteil, daß die Amplitudenänderung nur noch geringfügig vom Abstand abhängt; sie ist im Vergleich zur bekannten Paralleloptik um etwa den Faktor 10 kleiner.

Eine erhebliche Verbesserung läßt sich dadurch erreichen, daß die Meßsonde mit einem totalreflektierenden Spiegel im Form von zwei Glasprismen versehen ist, zwischen denen ein Luftspalt vorhanden ist. Durch den äußerst schmalen Luftspalt liegen Sende- und Empfangskanal optimal dicht aneinander. Außerdem ist der Aufbau der Meßsonde besonders einfach und kompakt. Der kompakte Aufbau der Meßsonde erlaubt deren Ausbildung zu einem separaten Bauteil, das durch ein biegsames Lichtleiterkabel an ein Grundgerät anschließbar ist, welches die Bauteile zur Erzeugung der Lichtimpulse, zur Umwandlung der empfangenen Lichtsignale in elektrische Impulssignale und zur Verarbeitung und Auswertung der elektrischen Impulssignale enthält. Die Meßsonde kann daher an einer anderen Stelle als das Grundgerät aufgestellt werden. Da in der Meßsonde und deren Lichtleiterkabel keine elektrischen Bauteile enthalten sind, ist eine Aufstellung in explosionsgefährdeten Räumen, Tankbehältern u. dgl. ohne weiteres möglich. Durch geeignete Maßnahmen besteht auch die Möglichkeit, an ein Grundgerät ein oder mehrer Meßsonden anzukuppeln.

Zum Schutze der Linse kann vor dieser ein Schutzrohr angeordnet sein; eine solche Ausbildung kann zweckmäßig sein, wenn z. B. in Tankbehältern die Gefahr von Spritzern o. dgl. besteht. Findet eine Messung nur in großen Zeitabständen statt, kann das Schutzrohr bedarfsweise auch mit einem Deckel ausgerüstet sein, der nur während der Meßphase geöffnet wird. Ein solcher Deckel läßt sich wegen seines exakt definierten Abstandes zur Optik auch zu Eichmessungen verwenden.

Da die Meßsonde über ein biegsames Lichtleiterkabel an das Grundgerät angeschlossen ist, läßt sie sich auch für eine kardanische Lagerung benutzen und mittels eines Stellmotors nach einem Steuerprogramm verstellen. Hierdurch ist beispielsweise das Abtasten von Schüttkegeln in Silos oder das Abtasten von Konturen möglich.

Zur Bildung einer homogenen Phasenfront der Lichtimpulse ist dem Sende-Lichtleiter im Grundgerät zweckmäßig zur Modenkopplung ein Vorlauf-Lichtleiter zugeordnet. Für Vergleichszwecke und zur Eichung kann im Sende-Lichtleiter und Empfangs-Lichtleiter je eine Lichtweiche zur Bildung eines kurzen Lichtweges für Referenzsignale angeordnet sein. Weiterhin kann es zweckmäßig sein, in dem Empfangs-Lichtleiter vor der Lichtweiche einen optischen Dämpfer anzuordnen, der mittels eines Stellmotors nach einem Steuerprogramm verstellbar ist und eine Anpassung an unterschiedliche Amplituden der Lichtimpulse bzw. an verschiedene Entfernungsbereiche erlaubt.

Der Gegenstand der Erfindung ist in der Zeichnung beispielsweise dargestellt; es zeigt:

Fig. 1 ein Grundgerät mit einer angekuppelten Meßsonde in einem Blockschaltbild,
Fig. 2 die Meßsonde der Fig. 1 in einer schematischen Darstellung und
Fig. 3 eine abgewandelte Meßsonde.

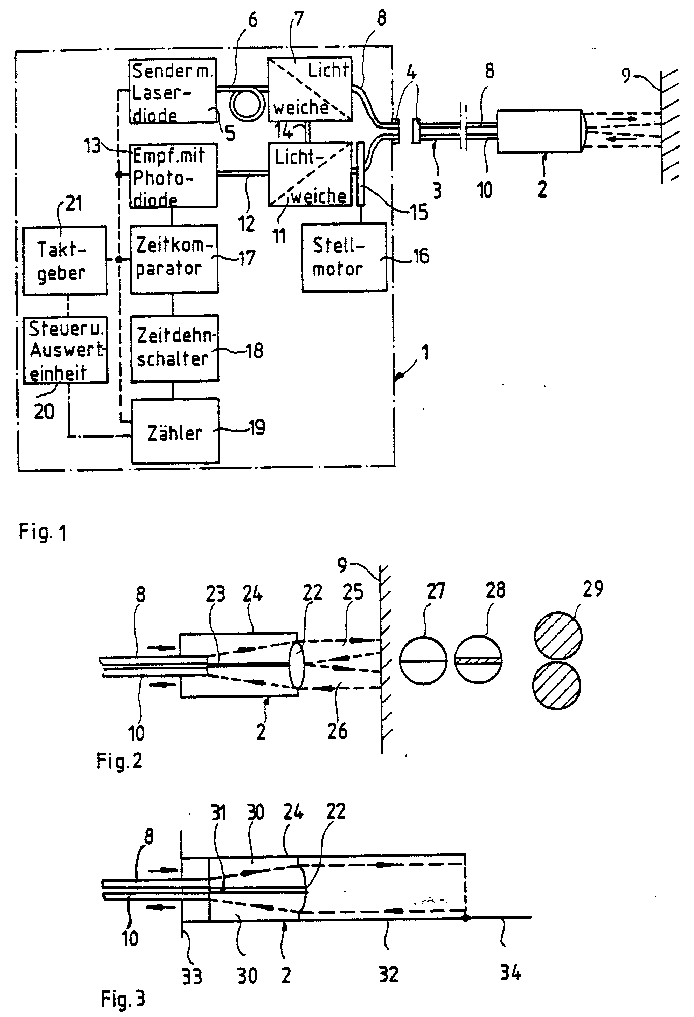

Das in Fig. 1 gezeigte optoelektrische Entfernungsmeßgerät besteht aus einem Grundgerät 1, an das eine Meßsonde 2 mittels eines biegsamen Lichtleiterkabels 3 und optischer Kupplungsorgane 4 angeschlossen ist. Das Grundgerät 1 erzeugt durch einen mit einer Laserdiode ausgerüsteten Sender 5 kurze Lichtimpulse, die zunächst zum Zwecke der Modenkopplung durch einen mehrere Meter langen Vorlauf-Lichtleiter 6 geführt werden, der in verschiedenen Richtungen, z. B. in der Form einer Acht gekrümmt angeordnet ist. Für eine ausreichende Modenkopplung hat sich eine Länge des Vorlauf-Lichtleiters 6 von 8 m als geeignet erwiesen. Vom Vorlauf-Lichtleiter 6 gelangen die Lichtimpulse über eine Lichtweiche 7, deren Bedeutung weiter unten erläutert wird, in den Sende-Lichtleiter 8, der aus einem dem Grundgerät 1 zugehörigen Abschnitt und einem dem biegsamen Lichtleiterkabel 3 zugehörigen Abschnitt besteht. Die Meßsonde 2 strahlt die Lichtimpulse gegen ein Zielobjekt 9 und nimmt die reflektierten Lichtsignale auf. Die aufgenommenen Lichtsignale werden über einen Empfangs-Lichtleiter 10, der ebenfalls aus zwei aneinanderkuppelbaren Abschnitten besteht, einer weiteren Lichtweiche 11 und einem Lichtleiter 12 einem mit einer Photodiode ausgerüsteten Empfänger 13 zugeführt, der Lichtsignale in elektrische Signale umwandelt. Die beiden erwähnten Lichtweichen 7 und 11 sind durch einen Referenz-Lichtleiter 14 verbunden. Durch die sendeseitige Lichtweiche 7 wird ein Teil der Lichtenergie, etwa 1 %, aus dem optischen Sendekanal in den Referenz-Lichtleiter 14 abgezweigt und durch die empfangsseitige Lichtweiche 11 in den optischen Empfangskanal geleitet. Dieses abgeleitete und über den kurzen Weg dem Empfangskanal zugeführte Lichtsignal bildet ein Referenzsignal, das von der Photodiode des Empfängers 13 zeitlich vor dem vom Zielobjekt reflektierten Lichtsignal (Zielsignal) empfangen wird, und zwar um die Zeit, die der Lichtimpuls zum Hin- und Rücklauf über den Sendelichtleiter 8, die Meßsonde 2 zum Zielobjekt 9 und zurück über die Meßsonde 2 sowie den Empfangs-Lichtleiter 10 benötigt. Aus Zielsignal und Referenzsignal läßt sich die Laufzeit des Zielumpulses und damit die Entfernung zwischen der Meßsonde 2 und dem Zielobjekt 9 bestimmen.

In dem Empfangs-Lichtleiter 10 kann bedarfsweise vor der Lichtweiche 11 ein optischer Dämpfer 15 angeordnet sein, der eine Anpassung an die Amplitudendynamik erlaubt. Dieser kann beispielsweise mittels eines Stellmotors 16 nach einem Steuerprogramm verstellbar sein. Stattdessen kann auch ein elektronisch steuerbares optisches Dämpfungsglied verwendet werden.

Die empfangenen Lichtsignale, nämlich die Referenz- und Zielsignale, werden mittels der Photodiode des Empfängers 13 in elektrische Impulse bzw. Signale umgewandelt und einem Zeitkomparator 17 zugeführt. Der Zeitkomparator 17 bildet Torimpulse (Rechteckimpulse) für einen Zähler 19, auf den innerhalb der Torzeit die Taktimpulse eines Taktgebers 21 (Quarzoszillator) aufgeschaltet werden. Hierfür werden die Torimpulse über einen Zeitdehnschalter 18 geführt, dem beispielsweise ein Zeitdehnfaktor von 500, 1000 o. dgl. zugeordnet sein kann. Diese Zeitdehnung erlaubt eine exakte Auszählung von Taktimpulsen, deren Frequenz z. B. in der Größenordnung von 50 MHz liegen kann.

Die gezählten, der Torzeit entsprechenden Taktimpulse werden zur Auswertung einer Steuer- und Auswertungseinheit 20 zugeführt. Der Taktgeber 21 triggert außerdem den Sender 5 und tastet wahlweise die Verstärkung des Empfängers 13. Weiterhin wird der Zeitkomparator 17 durch ein Zeitfenster angesteuert, welches die Verarbeitung des Referenzsignals oder des Zielsignals auswählt, wobei beispielsweise aufeinanderfolgend nur Referenzsignale oder nur Zielsignale verarbeitet werden können, um aus diesen Gruppen zu bilden, welche eine Auswertung durch Sortierung und Mittelwertbildung o. dgl. ermöglichen.

Die in Fig. 2 gezeigte Meßsonde 2 ist mit einer Linse 22 und einem doppelseitigen Spiegel 23 versehen, der längs der optischen Achse der Linse 22 bis etwa zum Brennpunkt verläuft, in dessen Bereich die Enden des Sende-Lichtleiters 8 und des Empfangs-Lichtleiters 10 nebeneinander angeordnet sind. Die genannten Bauteile sind in einem Gehäuse 24 fixiert. Durch diese Ausbildung ist eine Hälfte der Linse 22 dem Sendekanal 25 und die andere Hälfte dem Empfangskanal 26 zugeordnet. In der rechten Hälfte der Fig. 2 sind die Beleuchtungsfelder für verschiedene Abstände vor der Meßsonde gezeigt. Das für die Messung maßgebende Beleuchtungsfeld 27 bildet unmittelbar an der Linse 22 einen Strich. Das schraffierte Beleuchtungsfeld 28 gilt im Nahbereich und das Beleuchtungsfeld 29 im Fernbereich. Diese nur der Demonstration dienenden Beleuchtungsfelder zeigen, daß sich die Sende- und Empfangskanäle 25, 26 an der im Querschnitt breitesten Stelle überschneiden. Im Fernbereich werden die Aperturen der Enden der Sende- und Empfangs-Lichtleiter 8, 10 in der Gestalt einer Acht abgebildet.

Bei der Ausführung nach Fig. 3 ist die Meßsonde 2 mit einem totalreflektierenden Spiegel in Form von zwei Glasprismen 30, welche außerdem das optische Linsensystem beinhalten, versehen, zwischen denen sich ein Luftspalt 31 befindet. Weiterhin ist in Fig. 3 angedeutet, daß das Gehäuse 24 der Meßsonde 2 durch ein vor der Linse 22 befindliches Schutzrohr 32 verlängert sein kann. Für eine ortsfeste Anbringung an einem Tank, Silo o. dgl. können am Gehäuse 24 Flansche 33 vorgesehen sein. Auch kann das Schutzrohr 32 mit einem Deckel 34 ausgerüstet sein, der z. B. mittels eines Stellmotors (nicht dargestellt) nach einem Steuerprogramm verstellbar ist.

In Fig. 1 ist angedeutet, daß ein biegsames Lichtleiterkabel 3 über optische Kupplungsorgane 4 an den Sende- und Empfangs-Lichtleiter 8,10 des Grundgeräts 1 angeschlossen ist. Bedarfsweise können durch geeignete Lichtweichen oder optische Schalter am Grundgerät 1 auch mehrere Kupplungsstellen für zwei oder mehrere Meßsondenanschlüsse vorgesehen sein. Eine weitere Abwandlung läßt sich durch eine kardanische Lagerung der Meßsonde 2 schaffen, wobei diese mittels eines Stellmotors nach einem Steuerprogramm verstellbar ist.

## Patentansprüche

1. Optoelektronisches Entfernungsmeßgerät mit einem eine Laserdiode enthaltenden Lichtsender (5) zur Erzeugung kurzer Lichtimpulse, einem mit einer Photodiode ausgestatteten Photoempfänger (13), einer Meßsonde (2), welche eine Linsenoptik (22), einen Lichtimpulse der Linsenoptik zuführenden Sendelichterleiter (8) sowie einen Empfangslichtleiter (10) zur Weiterleitung der nach Reflexion an einem Zielobjekt über die Linsenoptik empfangenen Lichtimpulse auf die Photodiode aufweist, einer Auswerteschaltung, welche einen Taktgeber (21) sowie einen Zähler (19) zur Zählung der vom Taktgeber gelieferten Impulse während vorgegebener, durch die Aussendung und den Empfang der Lichtimpulse begrenzter Zeitintervalle enthält, dadurch gekennzeichnet, daß die Linsenoptik aus einer dem Sende- und dem Empfangsstrahlung gemensamen Linse (22) besteht, die der Linse (22) zugewandten Enden des Sende- und des Empfangslichtleiters (8, 10) im Bereich des Brennpunktes der Linse derart angeordnet sind, daß die optischen Achsen dieser Enden parallel zur optischen Achse der Linse verlaufen, die Meßsonde (2) einen doppelseitigen ebenen Spiegel (23) aufweist, der in einer die optische Achse der Linse enthaltenden Ebene liegt und im Bereich zwischen der Linse und den dieser zugewandten Enden der beiden Lichtleiter eine Trennfläche zwischen dem Sende- und dem Empfangsstrahlengang bildet.

2. Optoelektronisches Entfernungsmeßgerät nach Anspruch 1, dadurch gekennzeichnet, daß der Spiegel von einem zwischen zwei total reflektierenden Glasprismen (30) verlaufenden Luftspalt (31) gebildet ist.

3. Optoelektronisches Entfernungsmeßgerät nach Anspruch 1 oder 2, dadurch gekennzeichnet, daß die Meßsonde (2) als abtrennbares Bauteil ausgebildet und durch ein den Sende- und den Empfangslichtleiter (8, 10) enthaltendes biegsames Lichtleiterkabel (3) an ein Grundgerät (1) angeschlossen ist, welches den Lichtsender, den Photoempfänger und die Auswerte-Schaltung enthält.

4. Optoelektronisches Entfernungsmeßgerät nach Anspruch 3, dadurch gekennzeichnet, dan an das Grundgerät (1) weitere Meßsonden (2) ankuppelbar sind.

5. Optoelektronisches Entfernungsmeßgerät nach einem der Ansprüche 1 bis 4, dadurch gekennzeichnet, daß die Meßsonde (2) mit einem vor der Linse (22) angeordneten Schutzrohr (32) versehen ist.

6. Optoelektronisches Entfernungsmeßgerät nach Anspruch 5, dadurch gekennzeichnet, daß das Schutzrohr (32) mit einem Deckel (34) ausgerüstet ist.

7. Optoelektronisches Entfernungsmeßgerät nach einem der Ansprüche 1 bis 6, dadurch gekennzeichnet, daß die Meßsonde (2) kardanisch gelagert und mittels eines Stellmotors nach einem Steuerprogramm verstellbar ist.

8. Optoelektronisches Entfernungsmeßgerät nach einem der Ansprüche 4 bis 7, dadurch gekennzeichnet, daß dem Sende-Lichtleiter (8) im Grundgerät (1) zur Modenkupplung ein Vorlauf-Lichtleiter (6) zugeordnet ist.

9. Optoelektronisches Entfernungsmeßgerät nach einem der Ansprüche 1 bis 8, dadurch gekennzeichnet, daß im Sende-Lichtleiter (8) und Empfangs-Lichtleiter (10) je eine Lichtweiche (7, 11) zur Bildung eines kurzen Lichtweges für Referenzsignale angeordnet ist.

10. Optoelektronisches Entfernungsmeßgerät nach Anspruch 9, dadurch gekennzeichnet, daß in dem Empfangs-Lichtleiter (10) vor der Lichtweiche (11) ein optischer Dämpfer (15) angeordnet ist, der mittels eines Stellmotors (16) nach einem Steuerprogramm verstellbar ist.


**Claims**

1. Optoelectronic distance measuring apparatus with a light transmitter (5) containing a laser diode for generating short light pulses, a photodetector (13) equipped with a photodiode, a measuring probe (2) which comprises a lens system (22), a transmitting light guide (8) which transmits the light pulses to the lens system and a receiving light guide (10) for further transmission to the photodiode of the light pulses received via the lens system after reflection at a target object, and an analyser circuit which contains a clock generator (21) and a counter (19) for counting the pulses delivered by the clock generator during preset time intervals limited by transmission and reception of the light pulses, characterised in that the lens system consists of a lens (22) common to the paths of the transmitting and receiving beams, the ends of the transmitting and receiving light guides (8, 10) close to the lens (22) are arranged in the region of the focal point of the lens in such a way that the optical axes of these ends extend parallel to the optical axis of the lens, and the measuring probe (2) comprises a double-sided planar mirror (23) which is located in a plane containing the optical axis of the lens and, in the region between the lens and the ends of the two light guides close to the lens, forms a dividing plane between the paths of transmitting and receiving beams.

2. Optoelectrical distance measuring apparatus according to claim 1, characterised in that the mirror is formed by an air gap (31) extending between two totally reflecting glass prisms (30).

3. Optoelectrical distance measuring apparatus according to claim 1 or 2, characterised in that the measuring probe (2) is designed as a detachable component and connected by a flexible light guide cable (3) containing the transmitting and receiving light guides (8, 10) to a main apparatus (1) which contains the light transmitter, the photodetector and the analyser circuit.

4. Optoelectrical distance measuring apparatus according to claim 3, characterised in that additional measuring probes (2) can be coupled to the main apparatus (1).

5. Optoelectrical distance measuring apparatus according to any of claims 1 to 4, characterised in that the measuring probe (2) is provided with a protective tube (32) disposed in front of the lens (22).

6. Optoelectrical distance measuring apparatus according to claim 5, characterised in that the protective tube (32) is fitted with a cap (34).

7. Optoelectrical distance measuring apparatus according to any of claims 1 to 6, characterised in that the measuring probe (2) is gimbal mounted and displaceable according to a control programme by means of a servo motor.

8. Optoelectrical distance measuring apparatus according to any of claims 4 to 7, characterised in that a preliminary light guide (6) is associated with the transmitting light guide (8) in the main apparatus (1) for mode coupling.

9. Optoelectrical distance measuring apparatus according to any of claims 1 to 8, characterised in that in each of the transmitting light guide (8) and receiving light guide (10) is disposed a light deflector (7, 11) for forming a short light path for reference signals.

10. Optoelectrical distance measuring apparatus according to claim 9, characterised in that in the receiving light guide (10) in front of the light deflector (11) is disposed an optical damper (15) which is displaceable according to a control programme by means of a servo motor (16).


**Revendications**

1. Télémètre optoélectronique comportant une source de lumière (5) contenant une diode laser et servant à produire de brèves impulsions lumineuses, un photorécepteur (13) équipé d'une photodiode, une sonde de mesure (2), qui comporte un système optique à lentille (22), un guide de lumière d'émission (8), délivrant les impulsions lumineuses du système optique à lentille, ainsi qu'un guide de lumière de réception (10) pour la retransmission des impulsions lumineuses, reçues par l'intermédiaire du système optique à lentille après réflexion sur un objet-cible, à la photodiode, un circuit d'évaluation qui contient un générateur de cadence (21) ainsi qu'un compteur (19) servant à compter les impulsions délivrées par le généreteur de cadence, pendant un intervalle de temps prédéterminé, limité par l'émission et le réception des impulsions lumineuses, caractérisé par le fait que le système optique à lentille est constitué par une lentille (22) commune au trajet du rayonnement d'émission et au trajet du rayonnement de réception, que les extrémités, tournées vers la lentille (22), des guides de lumière d'émission et de réception (8, 10) sont disposées au voisinage du foyer de la lentille de telle sorte que les axes optiques de ces extrémités sont parallèles à l'axe optique de la lentille, et que la sonde de mesure (2) possède un miroir plan (23) à deux faces, qui est situé dans un plan passant par l'axe optique de la lentille et forme, dans la zone située entre la lentille et les extrémités, tournées vers cette dernière, des deux guides de lumière, une surface de séparation entre le trajet du rayonnement d'émission et le trajet du rayonnement de réception.

2. Télémètre optoélectrique suivant la revendication 1, caractérisé par le fait que le miroir est formé par une fente d'air (31), qui s'étend entre deux prismes en verre (30) à réflexion totale.

3. Télémètre optoélectrique suivant la revendication 1 ou 2, caractérisé par le fait que la sonde de mesure (2) est réalisée sous la forme d'un composant séparable et est raccordée, par un câble flexible (3) contenant les guides de lumière d'émission et de réception (8, 10), à un appareil de base (1), qui contient la source de lumière, le photorécepteur et le circuit d'évaluation.

4. Télémètre optoélectrique suivant la revendication 3, caractérisé par le fait que d'autres sondes de mesure (2) peuvent être accouplées à l'appareil de base (1).

5. Télémètre optoélectrique suivant l'une des revendications 1 à 4, caractérisé par le fait que la sonde de mesure (2) comporte un tube protecteur (32) disposé en avant de la lentille (22).

6. Télémètre optoélectrique suivant la revendication 5, caractérisé par le fait que le tube protecteur (32) est équipé d'un couvercle (34).

7. Télémètre optoélectrique suivant l'une des revendications 1 à 6, caractérisé par le fait que la sonde de mesure (2) est supportée selon un montage à la cardan et peut être réglée à l'aide d'un servomoteur conformément à un programme de commande.

8. Télémètre optoélectrique suivant l'une des revendications 4 à 7, caractérisé par le fait qu'un guide de lumière amont ( 6) servant à réaliser le couplage des modes, est associé au guide de lumière d'émission (8) dans l'appareil de base (1).

9. Télémètre optoélectrique suivant l'une des revendications 1 à 8, caractérisé par le fait que des aiguillages optiques (7, 11) sont disposés dans le guide de lumière d'émission (8) et dans le guide de lumière de réception (10), de manière à former un trajet court de déplacement de la lumière pour des signaux de référence.

10. Télémètre optoélectrique suivant la revendication 9, caractérisé par le fait que dans le guide de lumière de réception (10) se trouve disposé, en amont de l'aiguillage optique (11), un atténuateur optique, qui est réglable à l'aide d'un servomoteur (16) conformément à un programme de commande.

**Fig. 1**

Sender m. Laser-diode — 5
6
Licht weiche — 7
8
4
8 — 9
Empf. mit Photo-diode — 13
14
15
Licht-weiche — 11
3
10
2
12
Zeitkomparator — 17
Stell-motor — 16
Takt-geber — 21
Steuer u. Auswert. einheit — 20
Zeitdehn-schalter — 18
1
Zähler — 19

**Fig. 2**

8  23  24  22  25  9
10  2  26
27  28  29

**Fig. 3**

8  31  30  24  22
10
33  30  2  32  34